# EUROPEAN PATENT SPECIFICATION

(11) **EP 3 148 502 B1**
(45) Date of publication and mention of the grant of the patent: **11.03.2020**
(21) Application number: 15724698.4
(22) Date of filing: 28.05.2015
(51) Int. Cl.: A61K 8/36, A61K 8/37, A61K 8/55, A61Q 17/04, A61K 8/06

(54) **ANTI-SUN COMPOSITION IN THE FORM OF AN OIL-IN-WATER EMULSION COMPRISING AT LEAST ONE MIXTURE OF SURFACTANTS**
SONNENSCHUTZZUSAMMENSETZUNG IN FORM EINER ÖL-IN-WASSER-EMULSION MIT MINDESTENS EINEM TENSIDGEMISCH
COMPOSITION DE PROTECTION SOLAIRE SE PRÉSENTANT SOUS LA FORME D'UNE ÉMULSION HUILE DANS EAU CONTENANT AU MOINS UN MÉLANGE DE TENSIOACTIFS

(30) Priority: 28.05.2014 FR 1454854
(43) Date of publication of application: 05.04.2017
(73) Proprietor: L'OREAL, 75008 Paris (FR)
(72) Inventor: GROC, Marina, F-94152 Chevilly-Larue (FR); LALLORET, Florence, F-75014 Paris (FR); JOSSO, Martin, F-75007 Paris (FR); COTTARD, François, F-92400 Courbevoie (FR)
(74) Representative: Casalonga
(86) International application number: PCT/EP2015/061805
(87) International publication number: WO 2015/181276

(56) References cited:
- EP-A1- 2 921 157
- EP-A2- 2 616 038
- WO-A1-2014/097972
- WO-A1-2014/111562
- WO-A1-2014/111568
- WO-A1-2014/111574
- WO-A1-2014/191324
- WO-A1-2015/155158
- WO-A2-2014/111563
- WO-A2-2014/111566
- WO-A2-2014/111567
- WO-A2-2014/111569
- FR-A1- 2 983 716
- FR-A1- 2 983 718
- KR-A- 20110 078 358
- JANISTYN H: "UEBER AMPHISOL", PARFUMERIE UND KOSMETIK, HUETHIG, HEIDELBERG, DE, vol. 55, no. 2, 1 January 1974 (1974-01-01), pages 31-34, XP008030102, ISSN: 0031-1952

## Description

The present invention relates to a composition in the form of an oil-in-water emulsion comprising a) at least one continuous aqueous phase, b) at least one oily phase dispersed in said aqueous phase, c) at least one mixture of surfactants comprising i) one or more fatty acid esters of a polyalkylene glycol, ii) one or more fatty acid esters of glycerol, iii) one or more fatty acids in free form and iv) one or more alkali metal salts of an ester of phosphoric acid and of a fatty alcohol, and d) one or more UV-screening agents.

It is known that light radiation with wavelengths of between 280 nm and 400 nm permits tanning of the human epidermis and that rays with wavelengths more particularly between 280 and 320 nm, known as UVB rays, cause skin redness which can harm the development of a natural tan.

For these reasons, and also for aesthetic reasons, there is constant demand for means for controlling this natural tanning in order thus to control the colour of the skin; this UVB radiation should thus be screened out.

It is also known that UVA rays, with wavelengths between 320 and 400 nm, which cause tanning of the skin, are liable to induce adverse changes therein, in particular in the case of sensitive skin or skin that is continually exposed to solar radiation. UVA rays cause in particular a loss of elasticity of the skin and the appearance of wrinkles leading to premature ageing of the skin.

Thus, for aesthetic and cosmetic reasons, for instance conservation of the skin's natural elasticity, more and more people wish to control the effect of UVA rays on their skin. It is thus desirable also to screen out UVA radiation.

For the purpose of protecting the skin and keratin materials against UV radiation, cosmetic anti-sun compositions comprising UV-screening agents that are active with respect to the screening out of UVA and UVB radiation are generally used.

Among the various textures which currently exist, anti-sun compositions which are in the form of a fluid are generally highly liked by consumers since their fluidity allows them to be applied rapidly and easily over the whole of the body. In particular, these compositions in fluid form may be oil-in-water emulsions and may be sprayed onto the skin in the form of droplets of milk in order to obtain very safe tanning. By way of example, milk sprays are among the most fluid anti-sun compositions used, which can be deposited on the skin using an aerosol device or a spray pump device.

However, such compositions are generally difficult to spray, making their distribution on the skin usually not very uniform. Furthermore, these compositions need to be spread once again by the user in order to be sure to benefit from good protection against ultraviolet radiation.

The quality of spraying of a composition onto a surface is generally related to its viscosity, which should be as low as possible, which goes against its stability and its screening efficiency. Indeed, if the photoprotective composition is very fluid, then, on the one hand, it will be difficult to stabilize it and, on the other hand, it will result in an uneven deposit on the skin, which is not favourable to obtaining efficient protection against ultraviolet radiation.

Moreover, once sprayed, the compositions generally produce a relatively film-forming and tacky feel which is unliked by users.

Korean application KR 2011 0078358 disclosed sunscreen compositions in a form of emulsion with a low viscosity. However, these compositions were not entirely satisfactory, in particular in terms of stability for the case of extremely fluid compositions.

There is therefore a real need to provide compositions in the form of oil-in-water emulsions which do not have the abovementioned drawbacks, in particular which are stable and fluid while at the same time being capable of efficiently screening out ultraviolet radiation.

The applicant has discovered, surprisingly, that, by combining one or more fatty acid esters of a polyalkylene glycol, one or more fatty acid esters of glycerol, one or more fatty acids in free form and one or more alkali metal salts of an ester of phosphoric acid and of a fatty alcohol in a composition in the form of an oil-in-water emulsion comprising one or more UV-screening agents, it is possible to obtain an anti-sun composition having a very high sun protection factor (SPF), which is stable and extremely fluid.

A sun protection factor (SPF) which can reach a value of 50 is intended. The level of sun protection is defined by the sun protection factor (SPF) which is determined according to the "*in vitro*" method described by B. L. Diffey in J. Soc. Cosmet. Chem. 40, 127-133 (1989). The measurements were made using a UV-1000S spectrophotometer from the company Labsphere. Each composition is applied to a rough plate of PMMA, in the form of a homogeneous and even deposit in a proportion of 1 mg/cm².

The term "extremely fluid" is intended to mean that the viscosity of the compositions of the invention is less than or equal to 0.10 Pa.s at a temperature of 25°C, the viscosity being measured using a Rheomat 180 (from the company Lamy), equipped with an MS-R1, MS-R2, MS-R3, MS-R4 or MS-R5 spindle chosen according to the consistency of the composition, rotating at a rotation speed of 200 rpm.

The term "stable emulsions" is intended to mean emulsions which, after 2 months of storage at from 25°C to 45°C, do not exhibit any macroscopic change in colour, in odour or in viscosity, nor any variation in pH, and also no variation in microscopic appearance.

In other words, it is noted that the combination of surfactants used to stabilize the compositions in the form of an oil-in-water emulsion in accordance with the invention makes it possible to obtain photoprotective compositions which efficiently protect against UVA and UVB rays, and which are extremely fluid and stable.

Thus, the spraying qualities of the compositions according to the invention are improved by virtue of their considerable fluidity.

In particular, it is noted that the compositions according to the invention comprising such a combination of surfactants are more stable and more fluid and produce better performance levels in terms of sun protection factor than identical compositions not comprising the set of abovementioned surfactants.

In addition, the compositions according to the invention have good cosmetic properties and also exhibit good persistence with respect to water.

A subject of the present invention is in particular a composition in the form of an oil-in-water emulsion comprising:
a) a continuous aqueous phase,
b) an oily phase dispersed in said aqueous phase,
c) at least one mixture of surfactants comprising:
   i) one or more fatty acid esters of a polyalkylene glycol,
   ii) one or more fatty acid esters of glycerol,
   iii) one or more fatty acids in free form,
   iv) one or more alkali metal salts of an ester of phosphoric acid and of a fatty alcohol, and
d) one or more UV-screening agents;
said composition having a viscosity of less than or equal to 0.10 Pa.s at a temperature of 25°C, according to the measurement method described hereinafter.

The invention also relates to the use of the mixture of surfactants as described previously, for stabilizing a composition in the form of an oil-in-water emulsion containing one or more UV-screening agents.

The invention also relates to a non-therapeutic cosmetic process for limiting the darkening of the skin and/or improving the colour and/or uniformity of the complexion, comprising the application, to the surface of the skin, of at least one composition as defined previously.

The invention also relates to a non-therapeutic cosmetic process for preventing and/or treating the signs of ageing of a keratin material of at least one composition as defined previously.

Other subjects and characteristics, aspects and advantages of the invention will become even more clearly apparent on reading the description and the examples which follow.

Preferably, the composition comprises a physiologically acceptable medium and more preferentially a cosmetically acceptable medium.

For the purposes of the present invention, the term "physiologically acceptable medium" is intended to mean a medium that is suitable for the topical administration of a composition. A physiologically acceptable medium is preferentially a cosmetically or dermatologically acceptable medium, i.e. a medium that has no unpleasant odour or appearance, and that is entirely compatible with the topical administration route.

The viscosity of the compositions of the invention is measured using a Rheomat 180 (from the company Lamy), equipped with an MS-R1, MS-R2, MS-R3, MS-R4 or MS-R5 spindle chosen according to the consistency of the composition, rotating at a rotation speed of 200 rpm.

The term "fatty acid ester of a polyalkylene glycol" is intended to mean the esters of polyethylene glycol and of saturated or unsaturated fatty acids comprising from 12 to 30 carbon atoms, preferably from 14 to 26 carbon atoms, such as oleic alcohol or stearic acid, and comprising from 1 to 200 oxyethylene (OE) groups, preferably from 1 to 100 oxyethylene groups, such as polyethylene glycol 2 OE monostearate (INCI name: PEG-2 stearate) or polyethylene glycol 100 OE monostearate (INCI name: PEG-100 stearate). These polyethylene glycol esters may consists of 1 to 10 fatty chains.

Preferably, the fatty acid ester of a polyalkylene glycol of the compositions of the invention is PEG-100 stearate.

The fatty acid ester of a polyalkylene glycol may be present in a content ranging from 0.1% to 15% by weight relative to the total weight of the composition and preferably ranging from 0.5% to 5% by weight.

The term "fatty acid ester of glycerol" is intended to mean the esters of glycerol and of saturated or unsaturated fatty acids comprising from 12 to 30 carbon atoms, preferably from 14 to 26 carbon atoms, such as oleic alcohol or stearic acid, and comprising from 1 to 20 glyceryl groups, preferably from 1 to 10 glycerl units, such as glyceryl monostearate, glyceryl isostearate, diglyceryl distearate, tetraglyceryl tristearate, decaglyceryl decastearate, diglyceryl monostearate, hexaglyceryl tristearate, decaglyceryl pentastearate, the ester of glycerol and of palmitic and stearic acids, or glyceryl mono- and dibehenate.

Preferably, the fatty acid ester of glycerol of the compositions of the invention is chosen from glyceryl monostearate, glyceryl isostearate, and mixtures thereof.

Even more preferentially, the fatty acid ester of glycerol is a mixture of glyceryl monostearate and glyceryl isostearate.

The fatty acid ester of glycerol may be present in a content ranging from 0.1% to 15% by weight relative to the total weight of the composition and preferably from 0.5% to 5% by weight.

The term "fatty acid in free form" is intended to mean saturated or unsaturated fatty acids comprising from 12 to 30 carbon atoms, preferably from 14 to 26 carbon atoms, such as lauric acid, myristic acid, palmitic acid, stearic acid, arachidic acid, cetylic acid, cetearylic acid and behenic acid.

Preferably, the fatty acid in free form of the compositions of the invention is stearic acid.

The fatty acid in free form of the compositions of the invention may be present in a content ranging from 0.1% to 15% by weight relative to the total weight of the composition and preferably from 0.5% to 5% by weight.

The term "alkali metal salt of an ester of phosphoric acid and of a fatty alcohol" is intended to mean the alkali metal salts of esters of phosphoric acid and of a fatty alcohol comprising from 14 to 36 carbon atoms, preferably from 14 to 36 carbon atoms, such as cetyl alcohol or myristyl alcohol, and in particular:
- the sodium and potassium salts of monocetyl phosphate, for instance the compound sold under the reference Amphisol K by the company DSM,
- the alkali metal salts of dicetyl phosphate, and in particular the sodium and potassium salts,
- the alkali metal salts of dimyristyl phosphate, and in particular the sodium and potassium salts.

Preferably, the alkali metal salt of an ester of phosphoric acid and of a fatty alcohol of the compositions of the invention is the potassium salt of monocetyl phosphate.

The alkali metal salt of an ester of phosphoric acid and of a fatty alcohol of the compositions of the invention may be present in a content ranging from 0.1% to 15% by weight relative to the total weight of the composition and preferably from 0.5% to 5% by weight.

The term "high sun protection factor (SPF)" is intended to mean a sun protection factor (SPF) which can reach a value greater than or equal to 50. This range of high SPFs (greater than or equal to 50) is not, however, limiting for the compositions of the invention.

The level of sun protection is defined by the sun protection factor (SPF) which is determined according to the "*in vitro*" method described by B. L. Diffey in J. Soc. Cosmet. Chem. 40, 127-133 (1989). The measurements were made using a UV-1000S spectrophotometer from the company Labsphere. Each composition is applied to a rough plate of PMMA, in the form of a homogeneous and even deposit in a proportion of 1 mg/cm².

Preferably, the composition according to the invention has a sun protection factor value of between 6 and 70, preferably between 10 and 65 and even more preferably between 15 and 60.

Preferably, the composition according to the invention has a viscosity of less than 0.08 Pa.s and even more preferably less than 0.06 Pa.s at a temperature of 25°C, the viscosity being measured using a Rheomat 180 (from the company Lamy), equipped with an MS-R1, MS-R2, MS-R3, MS-R4 or MS-R5 spindle chosen according to the consistency of the composition, rotating at a rotation speed of 200 rpm.

The compositions according to the present invention also comprise one or more UV-screening agents in free form, chosen from water-soluble, liposoluble or insoluble organic UV-screening agents, and/or one or more inorganic pigments. It will preferentially consist of at least one hydrophilic, lipophilic or insoluble organic UV-screening agent.

The term "water-soluble UV-screening agent" is intended to mean any cosmetic or dermatological organic or inorganic compound for screening out UV radiation, which can be fully dissolved in molecular form in a liquid aqueous phase or else can be dissolved in colloidal form (for example in micellar form) in a liquid aqueous phase.

The term "liposoluble UV-screening agent" is intended to mean any cosmetic or dermatological organic or inorganic compound for screening out UV radiation, which can be fully dissolved in molecular form in a liquid fatty phase or else can be dissolved in colloidal form (for example in micellar form) in a liquid fatty phase.

The term "insoluble UV-screening agent" is intended to mean any cosmetic or dermatological organic or inorganic compound for screening out UV radiation which has a solubility in water of less than 0.5% by weight and a solubility of less than 0.5% by weight in the majority of organic solvents such as liquid paraffin, fatty alcohol benzoates and fatty acid triglycerides, for example Miglyol 812® sold by the company Dynamit Nobel. This solubility, determined at 70°C, is defined as the amount of product in solution in the solvent at equilibrium with an excess of solid in suspension after returning to ambient temperature. It may be readily evaluated in the laboratory.

The organic UV-screening agents in free form are chosen in particular from cinnamic compounds; anthranilate compounds; salicylic compounds; dibenzoylmethane compounds; benzylidenecamphor compounds; benzophenone compounds; β,β-diphenylacrylate compounds; triazine compounds; benzotriazole compounds; benzalmalonate compounds, in particular those cited in patent US 5 624 663; benzimidazole compounds; imidazoline compounds; bis-benzazolyl compounds, as described in patents EP 669 323 and US 2 463 264; p-aminobenzoic (PABA) compounds; methylenebis(hydroxyphenylbenzotriazole) compounds, as described in applications US 5 237 071, US 5 166 355, GB 2 303 549, DE 197 26 184 and EP 893 119; benzoxazole compounds, as described in patent applications EP 0 832 642, EP 1 027 883, EP 1 300 137 and DE 101 62 844; screening polymers and screening silicones, such as those described in particular in application WO 93/04665; α-alkylstyrene-based dimers, such as those described in patent application DE 198 55 649; 4,4-diarylbutadiene compounds, as described in applications EP 0 967 200, DE 197 46 654, DE 197 55 649, EP-A-1 008 586, EP 1 133 980 and EP 133 981; merocyanines as described in patent US 4 195 999, application WO 2004/006878, applications WO 2008/090066, WO 2011/113718 and WO 2009/027258, and the documents IP COM Journal No. 000179675D published on 23 February 2009, IP COM Journal No. 000182396D published on 29 April 2009, IP COM Journal No. 000189542D published on 12 November 2009 and IP COM Journal No. IPCOM000011179D published on 4 March 2004, and mixtures thereof.

As examples of organic photoprotective agents, mention may be made of those denoted hereinbelow under their INCI name:

### Cinnamic compounds:

Ethylhexyl methoxycinnamate sold in particular under the trade name Parsol MCX® by DSM Nutritional Products,
Isopropyl methoxycinnamate,
Isoamyl p-methoxycinnamate sold under the trade name Neo Heliopan E 1000® by Symrise,
DEA methoxycinnamate,
Diisopropyl methylcinnamate,
Glyceryl ethylhexanoate dimethoxycinnamate.

### Dibenzoylmethane compounds:

Butylmethoxydibenzoylmethane, sold in particular under the trade name Parsol 1789® by DSM Nutritional Products,
Isopropyldibenzoylmethane.

### para-Aminobenzoic compounds:

PABA,
Ethyl PABA,
Ethyl dihydroxypropyl PABA,
Ethylhexyl dimethyl PABA, sold in particular under the name Escalol 507® by ISP,
Glyceryl PABA,
PEG-25 PABA, sold under the name Uvinul P 25® by BASF.

### Salicylic compounds:

Homosalate, sold under the name Eusolex HMS® by Rona/EM Industries,
Ethylhexyl salicylate, sold under the name Neo Heliopan OS® by Symrise,
Dipropylene glycol salicylate, sold under the name Dipsal® by Scher,
TEA salicylate, sold under the name Neo Heliopan TS® by Symrise.

### β,β-Diphenylacrylate compounds:

Octocrylene, sold in particular under the trade name Uvinul N 539® by BASF,
Etocrylene, sold in particular under the trade name Uvinul N 35® by BASF.

### Benzophenone compounds:

Benzophenone-1, sold under the trade name Uvinul 400® by BASF,
Benzophenone-2, sold under the trade name Uvinul D 50® by BASF,
Benzophenone-3 or Oxybenzone, sold under the trade name Uvinul M 40® by BASF,
Benzophenone-4, sold under the trade name Uvinul MS 40® by BASF,
Benzophenone-5,
Benzophenone-6, sold under the trade name Helisorb 11® by Norquay,
Benzophenone-8, sold under the trade name Spectra-Sorb UV-24® by American Cyanamid,
Benzophenone-9, sold under the trade name Uvinul DS 49® by BASF,
Benzophenone-12,
n-Hexyl 2-(4-diethylamino-2-hydroxybenzoyl)benzoate, sold under the trade name Uvinul A Plus® or, as a mixture with octyl methoxycinnamate, under the trade name Uvinul A Plus B® by BASF,
1,1'-(1,4-Piperazinediyl)bis[1-[2-[4-(diethylamino)-2-hydroxybenzoyl]phenyl]methanone] (CAS 919803-06-8), such as described in patent application WO 2007/071 584; this compound advantageously being used in micronized form (mean size of 0.02 to 2 µm), which may be obtained, for example, according to the micronization process described in patent applications GB-A-2 303 549 and EP-A-893 119, and especially in the form of an aqueous dispersion.

### Benzylidenecamphor compounds:

3-Benzylidenecamphor, manufactured under the name Mexoryl SD® by Chimex,
4-Methylbenzylidenecamphor, sold under the name Eusolex 6300® by Merck,
Benzylidenecamphorsulfonic acid, manufactured under the name Mexoryl SL® by Chimex,
Camphor benzalkonium methosulfate, manufactured under the name Mexoryl SO® by Chimex,
Terephthalylidenedicamphorsulfonic acid, manufactured under the name Mexoryl SX® by Chimex,
Polyacrylamidomethylbenzylidenecamphor, manufactured under the name Mexoryl SW® by Chimex.

### Phenylbenzimidazole compounds:

Phenylbenzimidazolesulfonic acid, sold in particular under the trade name Eusolex 232® by Merck.

### Bis-benzazolyl compounds:

Disodium phenyl dibenzimidazole tetrasulfonate, sold under the trade name Neo Heliopan AP® by Symrise.

### Phenylbenzotriazole compounds:

Drometrizole trisiloxane, manufactured under the name Mexoryl XL® by Chimex.

### Methylenebis(hydroxyphenylbenzotriazole) compounds:

Methylenebis(benzotriazolyl)tetramethylbutylphenol, in particular in solid form, such as the product sold under the trade name Mixxim BB/100® by Fairmount Chemical, or in the form of an aqueous dispersion of micronized particles with a mean particle size ranging from 0.01 to 5 µm, more preferentially from 0.01 to 2 µm and more particularly from 0.020 to 2 µm, with at least one alkylpolyglycoside surfactant having the structure CₙH₂ₙ₊₁O(C₆H₁₀O₅)ₓH, in which n is an integer from 8 to 16 and x is the mean degree of polymerization of the (C₆H₁₀O₅) unit and ranges from 1.4 to 1.6, as described in patent GB-A-2 303 549, sold in particular under the trade name Tinosorb M® by the company BASF, or in the form of an aqueous dispersion of micronized particles with a mean particle size ranging from 0.02 to 2 µm, more preferentially from 0.01 to 1.5 µm and more particularly from 0.02 to 1 µm, in the presence of at least one polyglyceryl mono(C₈-C₂₀)alkyl ester with a degree of glycerol polymerization of at least 5, such as the aqueous dispersions described in patent application WO 2009/063 392.

### Triazine compounds:

- Bis(ethylhexyloxyphenol)methoxyphenyltriazine, sold under the trade name Tinosorb S® by BASF,
- Ethylhexyltriazone sold in particular under the trade name Uvinul T 150® by BASF,
- Diethylhexyl butamido triazone, sold under the trade name Uvasorb HEB® by Sigma 3V,
- 2,4,6-tris(dineopentyl 4'-aminobenzalmalonate)-s-triazine,
- 2,4,6-tris(diisobutyl 4'-aminobenzalmalonate)-s-triazine,
- 2,4-bis(n-butyl 4'-aminobenzoate)-6-(aminopropyltrisiloxane)-s-triazine,
- 2,4-bis(dineopentyl 4'-aminobenzalmalonate)-6-(n-butyl 4'-aminobenzoate)-s-triazine,
- symmetrical triazine screening agents substituted with naphthalenyl groups or polyphenyl groups described in patent US 6 225 467, patent application WO 2004/085 412 (see compounds 6 and 9) or the document "Symmetrical Triazine Derivatives", IP.COM IPCOM000031257 Journal, INC West Henrietta, NY, US (20 September 2004), in particular 2,4,6-tris(diphenyl)triazine and 2,4,6-tris(terphenyl)triazine, which is also mentioned in patent applications WO 06/035 000, WO 06/034 982, WO 06/034 991, WO 06/035 007, WO 2006/034 992 and WO 2006/034 985, these compounds advantageously being used in micronized form (mean particle size of 0.02 to 3 µm), which may be obtained, for example, according to the micronization process described in patent applications GB-A-2 303 549 and EP-A-893 119, and in particular in aqueous dispersion form,
- silicone triazines substituted with two aminobenzoate groups, as described in patent EP 0 841 341, in particular 2,4-bis(n-butyl 4'-aminobenzalmalonate)-6-[(3-{1,3,3,3-tetramethy1-1-[(trimethylsilyl)oxy]disiloxanyl}propyl)amino]-s-triazine.

### Anthranilic compounds:

Menthyl anthranilate, sold under the trade name Neo Heliopan MA® by Symrise.

### Imidazoline compounds:

Ethylhexyl dimethoxybenzylidene dioxoimidazoline propionate.

### Benzalmalonate compounds:

Polyorganosiloxane comprising benzalmalonate functional groups, such as Polysilicone-15, sold under the trade name Parsol SLX® by Hoffmann LaRoche.

### 4,4-Diarylbutadiene compounds:

- 1,1 -dicarboxy(2,2'-dimethylpropyl)-4,4-diphenylbutadiene.

### Benzoxazole compounds:

2,4-bis[5-(1,1-dimethylpropyl)benzoxazol-2-yl(4-phenyl)imino]-6-(2-ethylhexyl)imino-1,3,5-triazine, sold under the name Uvasorb K2A® by Sigma 3V.

The preferential organic screening agents in free form are chosen from:
Ethylhexyl methoxycinnamate,
Ethylhexyl salicylate,
Homosalate,
Butylmethoxydibenzoylmethane,
Octocrylene,
Phenylbenzimidazolesulfonic acid,
Benzophenone-3,
Benzophenone-4,
Benzophenone-5,
n-hexyl 2-(4-diethylamino-2-hydroxybenzoyl)benzoate,
4-Methylbenzylidenecamphor,
Terephthalylidenedicamphorsulfonic acid,
Disodium phenyl dibenzimidazole tetrasulfonate,
Methylenebis(benzotriazolyl)tetramethylbutylphenol,
Bis(ethylhexyloxyphenol)methoxyphenyltriazine
Ethylhexyl triazone,
Diethylhexyl butamido triazone,
2,4,6-tris(dineopentyl 4'-aminobenzalmalonate)-s-triazine,
2,4,6-tris(diisobutyl 4'-aminobenzalmalonate)-s-triazine,
2,4-bis(n-butyl 4'-aminobenzoate)-6-(aminopropyltrisiloxane)-s-triazine,
2,4-bis(dineopentyl 4'-aminobenzalmalonate)-6-(n-butyl 4'-aminobenzoate)-s-triazine,
Drometrizole trisiloxane,
Polysilicone-15,
1,1-dicarboxy(2,2'-dimethylpropyl)-4,4-diphenylbutadiene,
2,4-bis[5-(1,1-dimethylpropyl)benzoxazol-2-yl(4-phenyl)imino]-6-(2-ethylhexyl)imino-1,3,5-triazine,
and mixtures thereof.

The particularly preferred organic screening agents in free form are chosen from:
Ethylhexyl methoxycinnamate,
Ethylhexyl salicylate,
Homosalate,
Butylmethoxydibenzoylmethane,
Octocrylene,
Phenylbenzimidazolesulfonic acid,
n-hexyl 2-(4-diethylamino-2-hydroxybenzoyl)benzoate,
Terephthalylidenedicamphorsulfonic acid,
Methylenebis(benzotriazolyl)tetramethylbutylphenol,
2,4,6-tris(diphenyl)triazine,
Bis(ethylhexyloxyphenol)methoxyphenyltriazine,
Ethylhexyl triazone,
Diethylhexyl butamido triazone,
Drometrizole trisiloxane, and mixtures thereof.

The inorganic UV-screening agents used in accordance with the present invention are metal oxide pigments. More preferentially, the inorganic UV-screening agents of the invention are metal oxide particles with a mean elementary particle size of less than or equal to 0.5 µm, more preferentially between 0.005 and 0.5 µm, even more preferentially between 0.01 and 0.2 µm, better still between 0.01 and 0.1 µm and more particularly between 0.015 and 0.05 µm.

They may be chosen in particular from titanium oxide, zinc oxide, iron oxide, zirconium oxide and cerium oxide, or mixtures thereof.

Such coated or uncoated metal oxide pigments are described in particular in patent application EP-A-0 518 773. Commercial pigments that may be mentioned include the products sold by the companies Sachtleben Pigments, Tayca, Merck and Degussa.

The metal oxide pigments may be coated or uncoated.

The coated pigments are pigments that have undergone one or more surface treatments of chemical, electronic, mechanochemical and/or mechanical nature with compounds such as amino acids, beeswax, fatty acids, fatty alcohols, anionic surfactants, lecithins, sodium, potassium, zinc, iron or aluminium salts of fatty acids, metal alkoxides (of titanium or aluminium), polyethylene, silicones, proteins (collagen, elastin), alkanolamines, silicon oxides, metal oxides or sodium hexametaphosphate.

The coated pigments are more particularly titanium oxides that have been coated:
- with silica, such as the product Sunveil® from the company Ikeda,
- with silica and iron oxide, such as the product Sunveil F® from the company Ikeda,
- with silica and alumina, such as the products Microtitanium Dioxide MT 500 SA® and Microtitanium Dioxide MT 100 SA from the company Tayca and Tioveil from the company Tioxide,
- with alumina, such as the products Tipaque TTO-55 (B)® and Tipaque TTO-55 (A)® from the company Ishihara and UVT 14/4 from the company Sachtleben Pigments,
- with alumina and aluminium stearate, such as the products Microtitanium Dioxide MT 100 T®, MT 100 TX®, MT 100 Z® and MT-01® from the company Tayca, the products Solaveil CT-10 W® and Solaveil CT 100® from the company Uniqema and the product Eusolex T-AVO® from the company Merck,
   - with silica, alumina and alginic acid, such as the product MT-100 AQ® from the company Tayca,
   - with alumina and aluminium laurate, such as the product Microtitanium Dioxide MT 100 S® from the company Tayca,
   - with iron oxide and iron stearate, such as the product Microtitanium Dioxide MT 100 F® from the company Tayca,
   - with zinc oxide and zinc stearate, such as the product BR 351® from the company Tayca,
   - with silica and alumina and treated with a silicone, such as the products Microtitanium Dioxide MT 600 SAS®, Microtitanium Dioxide MT 500 SAS® or Microtitanium Dioxide MT 100 SAS® from the company Tayca,
   - with silica, alumina and aluminium stearate and treated with a silicone, such as the product STT-30-DS® from the company Titan Kogyo,
   - with silica and treated with a silicone, such as the product UV-Titan X 195® from the company Sachtleben Pigments,
   - with alumina and treated with a silicone, such as the products Tipaque TTO-55 (S)® from the company Ishihara or UV Titan M 262® from the company Sachtleben Pigments,
   - with triethanolamine, such as the product STT-65-S from the company Titan Kogyo,
   - with stearic acid, such as the product Tipaque TTO-55 (C)® from the company Ishihara,
   - with sodium hexametaphosphate, such as the product Microtitanium Dioxide MT 150 W® from the company Tayca,
   - TiO₂ treated with octyltrimethylsilane, sold under the trade name T 805® by the company Degussa Silices,
   - TiO₂ treated with a polydimethylsiloxane, sold under the trade name 70250 Cardre UF TiO2SI3® by the company Cardre,
   - anatase/rutile TiO₂ treated with a polydimethylhydrogenosiloxane, sold under the trade name Microtitanium Dioxyde USP Grade Hydrophobic® by the company Color Techniques.

Mention may also be made of TiO₂ pigments doped with at least one transition metal such as iron, zinc or manganese and more particularly manganese. Preferably, said doped pigments are in the form of an oily dispersion. The oil present in the oily dispersion is preferably chosen from triglycerides including those of capric/caprylic acids. The oily dispersion of titanium oxide particles may also comprise one or more dispersants, for instance a sorbitan ester, for instance sorbitan isostearate, or a polyoxyalkylenated fatty acid ester of glycerol, for instance TRI-PPG-3 myristyl ether citrate and polyglyceryl-3 polyricinoleate. Preferably, the oily dispersion of titanium oxide particles comprises at least one dispersant chosen from polyoxyalkylenated fatty acid esters of glycerol. Mention may be made more particularly of the oily dispersion of TiO₂ particles doped with manganese in capric/caprylic acid triglyceride in the presence of TRI-PPG-3 myristyl ether citrate and polyglyceryl-3 polyricinoleate and sorbitan isostearate having the INCI name: titanium dioxide (and) TRI-PPG-3 myristyl ether citrate (and) polyglyceryl-3 ricinoleate (and) sorbitan isostearate, for instance the product sold under the trade name Optisol TD50® by the company Croda.

The uncoated titanium oxide pigments are sold, for example, by the company Tayca under the trade names Microtitanium Dioxide MT 500 B or Microtitanium Dioxide MT 600 B®, by the company Degussa under the name P 25, by the company Wackherr under the name Transparent titanium oxide PW®, by the company Miyoshi Kasei under the name UFTR®, by the company Tomen under the name ITS® and by the company Tioxide under the name Tioveil AQ®.

The uncoated zinc oxide pigments are for example:
- those sold under the name Z-Cote by the company Sunsmart;
- those sold under the name Nanox® by the company Elementis;
- those sold under the name Nanogard WCD 2025® by the company Nanophase Technologies.

The coated zinc oxide pigments are for example:
- those sold under the name Zinc Oxide CS-5® by the company Toshibi (ZnO coated with polymethylhydrogenosiloxane);
- those sold under the name Nanogard Zinc Oxide FN® by the company Nanophase Technologies (as a 40% dispersion in Finsolv TN®, C12-C15 alkyl benzoate);
- those sold under the name Daitopersion Zn-30® and Daitopersion Zn-50® by the company Daito (dispersions in cyclopolymethylsiloxane/oxyethylenated polydimethylsiloxane, containing 30% or 50% of zinc oxides coated with silica and polymethylhydrogenosiloxane);
- those sold under the name NFD Ultrafine ZnO® by the company Daikin (ZnO coated with perfluoroalkyl phosphate and copolymer based on perfluoroalkylethyl as a dispersion in cyclopentasiloxane);
- those sold under the name SPD-Z1® by the company Shin-Etsu (ZnO coated with silicone-grafted acrylic polymer, dispersed in cyclodimethylsiloxane);
- those sold under the name Escalol Z100® by the company ISP (alumina-treated ZnO dispersed in an ethylhexyl methoxycinnamate/PVP-hexadecene/methicone copolymer mixture);
- those sold under the name Fuji ZnO-SMS-10® by the company Fuji Pigment (ZnO coated with silica and polymethylsilsesquioxane);
- those sold under the name Nanox Gel TN® by the company Elementis (ZnO dispersed at a concentration of 55% in C₁₂-C₁₅ alkyl benzoate with hydroxystearic acid polycondensate).

The uncoated cerium oxide pigments may be, for example, those sold under the name Colloidal Cerium Oxide® by the company Rhône-Poulenc.

The uncoated iron oxide pigments are sold, for example, by the company Arnaud under the names Nanogard WCD 2002® (FE 45B®), Nanogard Iron FE 45 BL AQ®, Nanogard FE 45R AQ® and Nanogard WCD 2006® (FE 45R®) or by the company Mitsubishi under the name TY-220®.

The coated iron oxide pigments are sold, for example, by the company Arnaud under the names Nanogard WCD 2008 (FE 45B FN)®, Nanogard WCD 2009® (FE 45B 556®), Nanogard FE 45 BL 345® and Nanogard FE 45 BL® or by the company BASF under the name Transparent Iron Oxide®.

Mention may also be made of mixtures of metal oxides, in particular of titanium dioxide and of cerium dioxide, including the equal-weight mixture of titanium dioxide and cerium dioxide coated with silica, sold by the company Ikeda under the name Sunveil A®, and also the mixture of titanium dioxide and zinc dioxide coated with alumina, silica and silicone, such as the product M 261® sold by the company Sachtleben Pigments, or coated with alumina, silica and glycerol, such as the product M 211® sold by the company Sachtleben Pigments.

According to the invention, coated or uncoated titanium oxide pigments are particularly preferred.

The UV-screening agents according to the invention are preferably present in the compositions according to the invention in a content ranging from 0.1% to 45% by weight and in particular from 5% to 30% by weight relative to the total weight of the composition.

The compositions according to the invention are oil-in-water emulsions. The emulsification processes that may be used are of the paddle or propeller and rotor-stator type.

As indicated previously, the compositions according to the invention comprise at least one continuous aqueous phase.

The aqueous phase contains water and optionally other water-soluble or water-miscible organic solvents.

An aqueous phase that is suitable for use in the invention may comprise, for example, a water chosen from a natural spring water, such as water from La Roche-Posay, water from Vittel or waters from Vichy, or a floral water.

The water-soluble or water-miscible solvents that are suitable for use in the invention comprise short-chain monoalcohols, for example C₁-C₄ monoalcohols, such as ethanol or isopropanol; diols or polyols, such as ethylene glycol, 1,2-propylene glycol, 1,3-butylene glycol, hexylene glycol, diethylene glycol, dipropylene glycol, 2-ethoxyethanol, diethylene glycol monomethyl ether, triethylene glycol monomethyl ether, glycerol and sorbitol, and mixtures thereof.

According to a preferred embodiment, use may more particularly be made of ethanol, propylene glycol, glycerol, and mixtures thereof.

According to one particular form of the invention, the overall aqueous phase, including all the hydrophilic substances of the composition capable of being dissolved in this same phase, may represent from 5% to 95% by weight and preferentially from 10% to 80% by weight, relative to the total weight of the composition.

According to the invention, the compositions of the invention do not comprise any aqueous-phase-thickening or -gelling polymer.

As indicated above, the composition according to the invention comprises at least one oily phase dispersed in the aqueous phase.

For the purposes of the invention, the term "oily phase" is intended to mean a phase comprising at least one oil and all of the liposoluble and lipophilic ingredients and the fatty substances used for the formulation of the compositions of the invention.

The term "oil" is intended to mean any fatty substance that is in liquid form at ambient temperature (20-25°C) and at atmospheric pressure (760 mmHg).

An oil that is suitable for use in the invention may be volatile or non-volatile.

An oil that is suitable for use in the invention may be chosen from hydrocarbon-based oils, silicone oils and fluoro oils, and mixtures thereof.

A hydrocarbon-based oil that is suitable for use in the invention may be an animal hydrocarbon-based oil, a plant hydrocarbon-based oil, a mineral hydrocarbon-based oil or a synthetic hydrocarbon-based oil.

An oil that is suitable for use in the invention may be advantageously chosen from mineral hydrocarbon-based oils, plant hydrocarbon-based oils, synthetic hydrocarbon-based oils and silicone oils, and mixtures thereof.

For the purposes of the present invention, the term "silicone oil" is intended to mean an oil comprising at least one silicon atom, and in particular at least one Si-O group.

The term "hydrocarbon-based oil" is intended to mean an oil containing mainly hydrogen and carbon atoms.

The term "fluoro oil" is intended to mean an oil comprising at least one fluorine atom.

A hydrocarbon-based oil that is suitable for use in the invention may also optionally comprise oxygen, nitrogen, sulfur and/or phosphorus atoms, for example in the form of hydroxyl, amine, amide, ester, ether or acid groups, and in particular in the form of hydroxyl, ester, ether or acid groups.

The oily phase may comprise one or more volatile or non-volatile hydrocarbon-based oils and/or one or more volatile and/or non-volatile silicone oils.

For the purposes of the invention, the term "volatile oil" is intended to mean an oil that is capable of evaporating on contact with the skin or the keratin fibre in less than one hour, at ambient temperature and atmospheric pressure. The volatile oil(s) of the invention are volatile cosmetic oils which are liquid at ambient temperature and which have a non-zero vapour pressure, at ambient temperature and atmospheric pressure, ranging in particular from 0.13 Pa to 40 000 Pa (10⁻³ to 300 mmHg), in particular ranging from 1.3 Pa to 13 000 Pa (0.01 to 100 mmHg) and more particularly ranging from 1.3 Pa to 1300 Pa (0.01 to 10 mmHg).

The term "non-volatile oil" is intended to mean an oil that remains on the skin or the keratin fibre at ambient temperature and atmospheric pressure for at least several hours, and that especially has a vapour pressure of less than 10⁻³ mmHg (0.13 Pa).

Among the non-volatile hydrocarbon-based oils that can be used according to the invention, mention may be made of glyceride triesters and in particular caprylic/capric acid triglycerides such as those sold by the company Stearineries Dubois or those sold under the names Miglyol 810®, 812® and 818® by the company Dynamit Nobel, fatty amides such as isopropyl N-lauroyl sarcosinate, such as the product sold under the trade name Eldew SL 205® from Ajinomoto, synthetic esters, and in particular isononyl isononanoate, diisopropyl sebacate, C₁₂-C₁₅ alkyl benzoate, such as the product sold under the trade name Finsolv TN® or Witconol TN® by the company Witco or Tegosoft TN ® by the company Evonik Goldschmidt, 2-ethylphenylbenzoate, such as the commercial product sold under the name X-Tend 226® by the company ISP, and fatty alcohols, in particular octyldodecanol.

As volatile hydrocarbon-based oils that may be used according to the invention, mention may be made in particular of hydrocarbon-based oils having from 8 to 16 carbon atoms and in particular of branched C₈-C₁₆ alkanes, such as C₈-C₁₆ isoalkanes of petroleum origin (also known as isoparaffins), such as isododecane (also known as 2,2,4,4,6-pentamethylheptane), isodecane or isohexadecane, the oils sold under the Isopar or Permethyl trade names, branched C₈-C₁₆ esters, isohexyl neopentanoate, and mixtures thereof.

Among the non-volatile silicone oils, mention may be made of non-volatile polydimethylsiloxanes (PDMSs), polydimethylsiloxanes comprising alkyl or alkoxy groups which are pendent and/or at the end of the silicone chain, which groups each contain from 2 to 24 carbon atoms, or phenyl silicones, such as phenyl trimethicones, phenyl dimethicones, phenyl(trimethylsiloxy)diphenylsiloxanes, diphenyl dimethicones, diphenyl(methyldiphenyl)trisiloxanes or (2-phenylethyl)trimethylsiloxysilicates.

Among the volatile silicone oils, mention may for example be made of volatile linear or cyclic silicone oils, especially those with a viscosity ≤ 8 centistokes (8 × 10⁻⁶ m²/s) and especially containing from 2 to 7 silicon atoms, these silicones optionally comprising alkyl or alkoxy groups containing from 1 to 10 carbon atoms. As volatile silicone oil that may be used in the invention, mention may be made in particular of octamethylcyclotetrasiloxane, decamethylcyclopentasiloxane, dodecamethylcyclohexasiloxane, heptamethylhexyltrisiloxane, heptamethyloctyltrisiloxane, hexamethyldisiloxane, octamethyltrisiloxane, decamethyltetrasiloxane and dodecamethylpentasiloxane, and mixtures thereof.

Among the volatile fluoro oils, mention may be made of nonafluoromethoxybutane, decafluoropentane, tetradecafluorohexane and dodecafluoropentane, and mixtures thereof.

An oily phase according to the invention may also comprise other fatty substances, mixed with or dissolved in the oil.

Another fatty substance that may be present in the oily phase may be, for example:
- a fatty acid other than stearic acid, chosen from fatty acids comprising from 8 to 30 carbon atoms, such as lauric acid, palmitic acid and oleic acid;
- a wax chosen from waxes such as lanolin, beeswax, carnauba or candelilla wax, paraffin waxes, lignite waxes, microcrystalline waxes, ceresin or ozokerite, or synthetic waxes, such as polyethylene waxes or Fischer-Tropsch waxes;
- a gum chosen from silicone gums (dimethiconol);
- a pasty compound, such as polymeric or non-polymeric silicone compounds, esters of a glycerol oligomer, arachidyl propionate, fatty acid triglycerides and derivatives thereof;
- and mixtures thereof.

The compositions of the invention preferably comprise raw materials which do not themselves filter out UV radiation, but make it possible to amplify the screening performance of the compositions of the invention: these are efficiency boosters. Mention is for example made of the particles of styrene/alkyl acrylate copolymer sold under the name Sunspheres Powder by Dow Chemical; mention is also made of waxes such as polymethylene wax (Cirebelle 303 sold by the company Cirebelle). Mention is also made of gelling lipophilic polymers such as the semicrystalline polyacrylates sold under the names Intelimer IPA 13-1, Intelimer IPA 13-6 or Uniclear 100 VG by the company Air Products and Chemicals, or else Uniclear 100 VG-PA.

The compositions of the invention may contain any of the additives normally used in cosmetics and will find uses in the field of anti-sun, care and makeup products.

The aqueous phase may contain glycols, such as dipropylene glycol, glycerol and butylene glycol, active agents, salts, fillers, organic particles, synthetic neutral polymers such as N-vinylpyrrolidone, water-soluble or water-dispersible silicone derivatives, such as acrylic silicones, silicone polyethers and cationic silicones.

As active agents, use may in particular be made of vitamins (A, C, E, K, PP, etc), alone or as a mixture, and also derivatives thereof, keratolytic and/or desquamating agents (salicylic acid and derivatives thereof, alpha-hydroxy acids, ascorbic acid and derivatives thereof), anti-inflammatories, calmatives, depigmenting agents, tensioning agents such as synthetic polymers, plant proteins, wax dispersions, mixed silicates and colloidal particles of inorganic fillers; matting agents, agents for preventing hair loss and/or hair restorers, or else anti-wrinkle agents, and mixtures thereof.

The oily phase may contain lipophilic gelling agents, surfactants, waxes, organic or inorganic particles and, in addition to the polar oil(s) of the invention, oils of alkane, silicone or fluoro type.

The present invention also relates to the use of the mixture of surfactants as described previously, for stabilizing a composition in the form of an oil-in-water emulsion containing one or more UV-screening agents.

The examples that follow serve to illustrate the invention.

### EXAMPLES

### I. Compositions tested

The compositions (A) to (A5) are prepared, the amounts of which are expressed as percentage by weight unless otherwise indicated.

| Phase | | A (invention) | A1 | A2 | A3 | A4 | A5 |
|---|---|---|---|---|---|---|---|
| I | Glycerol | 6 | 6 | 6 | 6 | 6 | 6 |
| | Complexing agent | 0.1 | 0.1 | 0.1 | 0.1 | 0.1 | 0.1 |
| | Propylene glycol | 3 | 3 | 3 | 3 | 3 | 3 |
| | Styrene/acrylate copolymer (Sunspheres) | - | - | - | - | - | - |
| | Mexoryl SX | 0.3 am | 0.3 am | 0.3 am | 0.3 am | 0.3 am | 0.3 am |
| | Triethano lamine | 0.3 | 0.3 | 0.3 | 0.3 | 0.3 | 0.3 |
| | Preserving agents | 1.2 | 1.2 | 1.2 | 1.2 | 1.2 | 1.2 |
| | Deionized water | qs 100 | qs 100 | qs 100 | qs 100 | qs 100 | qs 100 |
| II | Preserving agents | 0.25 | 0.25 | 0.25 | 0.25 | 0.25 | 0.25 |
| | **PEG 100 stearate** | **0.9** | - | **0.9** | **0.9** | **0.9** | **0.9** |
| | **Glyceryl monostearate** | **0.9** | **0.9** | - | **0.9** | **0.9** | **0.9** |
| | **Stearic acid** | **0.9** | **0.9** | **0.9** | - | **0.9** | **0.9** |
| | **Monocetyl phosphate** | **1** | **1** | **1** | 1 | - | **1** |
| | **Glyceryl isostearate** | **1** | **1** | **1** | **1** | **1** | - |
| | Synthetic wax | 1 | 1 | 1 | 1 | 1 | 1 |
| | Octocrylene | 5 | 5 | 5 | 5 | 5 | 5 |
| | Bis-ethylhexyloxyloxy-phenol-methoxy-phenyltriazine | - | - | - | - | - | - |
| | 4-tert-butyl-4'-methoxy dibenzoyl methane | 5 | 5 | 5 | 5 | 5 | 5 |
| | Drometrizole trisiloxane | - | - | - | - | - | - |
| | Ethylhexyl salicylate | 5 | 5 | 5 | 5 | 5 | 5 |
| | Homosolate | 7 | 7 | 7 | 7 | 7 | 7 |
| | Ethylhexyltriazone | 5 | 5 | 5 | 5 | 5 | 5 |
| | Titanium dioxide | 2.5 | 2.5 | 2.5 | 2.5 | 2.5 | 2.5 |
| III | Acrylate copolymer Poly(stearyl acrylate) Intelimer 13-1 | - | - | - | - | - | - |
| IV | Ethanol | 4 | 4 | 4 | 4 | 4 | 4 |

The following compositions (B) and (C) according to the invention are also prepared, the amounts of which are expressed as percentage by weight unless otherwise indicated.

| Phase | | B (invention) | C (invention) |
|---|---|---|---|
| I | Glycerol | 6 | 6 |
| | Complexing agent | 0.1 | 0.1 |
| | Propylene glycol | - | 3 |
| | Styrene/acrylate copolymer (Sunspheres) | 4 | 4 |
| | Mexoryl SX | - | 0.3% am |
| | Triethanolamine | 0.3 | 0.3 |
| | Preserving agents | 1.2 | 1.2 |
| | Deionized water | qs 100 | qs 100 |
| II | Preserving agents | 0.25 | 0.25 |
| | **PEG 100 stearate** | **0.9** | **0.9** |
| | **Glyceryl monostearate** | **0.9** | 0.9 |
| | **Stearic acid** | **0.9** | **0.9** |
| | **Monocetyl phosphate** | **1** | **1** |
| | **Glyceryl isostearate** | **1** | **1** |
| | Synthetic wax | - | 1 |
| | Octocrylene | 5 | 5 |
| | Bis-ethylhexyloxyloxy-phenolmethoxyphenyltriazine | - | 1.4 |
| | 4-tert-butyl-4' - methoxydibenzoyl methane | 5 | 5 |
| | Drometrizole trisiloxane | 0.55 | 1 |
| | Ethylhexyl salicylate | 5 | 5 |
| | Homosolate | 10 | 7 |
| | Ethylhexyltriazone | 2.5 | 2 |
| | Titanium dioxide | 1 | - |
| III | Acrylate copolymer Poly(stearyl acrylate) Intelimer 13-1 | 0.15 | - |
| IV | Ethanol | 4 | 4 |

### II. Procedure for the compositions

The aqueous phase (I) and the oily phase (II) are prepared by mixing the ingredients with mechanical stirring at a temperature of 80°C; the solutions obtained are macroscopically homogeneous.

The emulsion is prepared by slowly introducing the oily phase (II) into the aqueous phase (I) with stirring using a homogenizer of COS 1000 type at a speed of 4000 rpm for 15 minutes. The emulsion obtained is cooled, with stirring, to 40°C and then the phase (III) is added with gentle stirring, followed by the phase (IV).

The emulsion obtained is cooled to ambient temperature with slow stirring. The emulsion is characterized by drops having a size of between 500 nanometres and 10 µm and has a viscosity of less than 100 mPa.s.

### III. Protocol for evaluating in vitro the screening efficiency

The sun protection factor (SPF) is determined according to the "*in vitro*" method described by B. L. Diffey in J. Soc. Cosmet. Chem. 40, 127-133 (1989). The measurements were made using a UV-1000S spectrophotometer from the company Labsphere. Each composition is applied to a rough plate of PMMA, in the form of a homogeneous and even deposit in a proportion of 1 mg/cm².

### III. Protocol for evaluating the stability of the compositions

The stability of the compositions of the invention is evaluated by macroscopic and microscopic observations of their appearance and by measuring their viscosity.

A composition is judged to be stable when its macroscopic appearance and microscopic appearance, its pH and its viscosity do not vary for 2 months at from 25°C to 45°C.

### IV. Protocol for evaluating the viscosity of the emulsions

The viscosity of the compositions is measured using a Rheomat 180 (from the company Lamy), equipped with an MS-R1, MS-R2, MS-R3, MS-R4 or MS-R5 spindle chosen according to the consistency of the composition, rotating at a rotation speed of 200 rpm.

### VI. Results

| Examples | *in vitro* SPF | Stability | Viscosity (mPa.s) |
|---|---|---|---|
| A | 78 | ++++ | 10.6 |
| B | 73 | ++++ | 62 |
| C | 75 | ++++ | 21 |
| A1 | not measurable | Setting after 1 week at 25°C | 25.8 |
| A2 | not measurable | Heterogeneous appearance after 24 h - Aggregates | 13.6 |
| A3 | 36 | Heterogeneous appearance after 2 months at 25°C Aggregates | 11 |
| A4 | not measurable | Very heterogeneous appearance after 24 h - Aggregates | Not measurable |
| A5 | 54 | Heterogeneous appearance after 2 months at 45°C | 19.8 |

No change in terms of the macroscopic and microscopic appearances and in terms of viscosity is noted after two months of storage at from 25°C to 45°C for compositions A, B and C.

These results show that only the combination of at least one fatty acid ester of a polyalkylene glycol (PEG-100 stearate), at least one fatty acid ester of glycerol (i.e.: mixture of glyceryl monostearate and glyceryl isostearate), at least one fatty acid in free form (i.e. stearic acid) and at least one alkali metal salt of an ester of phosphoric acid and of a fatty alcohol makes it possible to obtain an oil-in-water emulsion which is stable and extremely fluid and has a high SPF protection level.

For the combinations of surfactants not comprising the collection of surfactants of the invention, the performance levels in terms of screening efficiency, stability and viscosity are inferior to those of the combination of the composition according to the invention.

## Claims

1. Composition in the form of an oil-in-water emulsion comprising:
a) a continuous aqueous phase,
b) an oily phase dispersed in said aqueous phase,
c) at least one mixture of surfactants comprising:
i) one or more fatty acid esters of a polyalkylene glycol,
ii) one or more fatty acid esters of glycerol,
iii) one or more fatty acids in free form,
iv) one or more alkali metal salts of an ester of phosphoric acid and of a fatty alcohol, and
d) one or more UV-screening agents;
said composition has a viscosity of less than or equal to 0.10 Pa.s at a temperature of 25°C, the viscosity being measured using a Rheomat 180, equipped with an MS-R1, MS-R2, MS-R3, MS-R4 or MS-R5 spindle chosen according to the consistency of the composition, rotating at a rotation speed of 200 rpm; and
said composition does not comprise any aqueous-phase-thickening or -gelling polymer; and
wherein:
- said fatty acids in fatty acid esters of a polyalkylene glycol i), fatty acid esters of glycerol ii) and fatty acids in free form iii) are, independently of each other, saturated or unsaturated fatty acids comprising from 12 to 30 carbon atoms, and
- said fatty alcohol(s) in alkali metal salts of an ester of phosphoric acid and of a fatty alcohol iv) comprise(s) from 14 to 36 carbon atom.

2. Composition according to Claim 1, **characterized in that** the fatty acid ester of polyethylene glycol is an ester of polyethylene glycol comprising from 1 to 200 oxyethylene groups, preferably from 1 to 100 oxyethylene groups, and of a saturated or unsaturated fatty acid comprising from 14 to 26 carbon atoms.

3. Composition according to Claim 1 or 2, **characterized in that** the fatty acid ester of polyethylene glycol is chosen from polyethylene glycol 2 OE monostearate or polyethylene glycol 100 monostearate.

4. Composition according to any one of the preceding claims, **characterized in that** the fatty acid ester of polyethylene glycol is PEG-100 stearate.

5. Composition according to any one of the preceding claims, **characterized in that** the fatty acid ester of glycerol is an ester of a saturated or unsaturated fatty acid comprising from 14 to 26 carbon atoms, and from 1 to 20 glyceryl groups and preferably from 1 to 10 glyceryl units.

6. Composition according to any one of the preceding claims, **characterized in that** the fatty acid ester of glycerol is chosen from glyceryl monostearate, glyceryl isostearate, diglyceryl distearate, tetraglyceryl tristearate, decaglyceryl decastearate, diglyceryl monostearate, hexaglyceryl tristearate, decaglyceryl pentastearate, the ester of glycerol and of palmitic and stearic acids, glyceryl mono- and dibehenate, and mixtures thereof.

7. Composition according to Claim 6, **characterized in that** the fatty acid ester of glycerol is chosen from glyceryl monostearate, glyceryl isostearate, and mixtures thereof.

8. Composition according to any one of the preceding claims, **characterized in that** the fatty acid is chosen from lauric acid, myristic acid, palmitic acid, stearic acid, arachidic acid, cetylic acid, cetearylic acid and behenic acid.

9. Composition according to any one of the preceding claims, **characterized in that** the fatty acid in free form is stearic acid.

10. Composition according to any one of the preceding claims, **characterized in that** the alkali metal salts of esters of phosphoric acid and of a fatty alcohol are chosen from the sodium and potassium salts of monocetyl phosphate, the alkali metal salts of dicetyl phosphate and the alkali metal salts of dimyristyl phosphate.

11. Composition according to any one of the preceding claims, **characterized in that** the alkali metal salt of an ester of phosphoric acid and of a fatty alcohol is the potassium salt of monocetyl phosphate.

12. Composition according to any one of the preceding claims, **characterized in that** the viscosity of the composition is less than or equal to 0.08 Pa.s and even more preferably less than 0.06 Pa.s at a temperature of 25°C.

13. Composition according to any one of the preceding claims, **characterized in that** the screening agent(s) are UV-screening agents in free form, chosen from hydrophilic, lipophilic or insoluble organic UV-screening agents, and/or one or more inorganic pigments.

14. Composition according to any one of the preceding claims, **characterized in that** it comprises a physiologically acceptable medium and more preferentially a cosmetically acceptable medium.

15. Use of the mixture of surfactants c) as defined in Claim 1, for stabilizing a composition in the form of an oil-in-water emulsion containing one or more UV-screening agents.

16. Composition according to any one of claims 1 to 14 for its use in a method for limiting the darkening of the skin and/or improving the colour and/or uniformity of the complexion.

17. Composition according to any one of claims 1 to 14 for its use in a method for preventing and/or treating the signs of ageing of a keratin material, comprising the application.

## Patentansprüche

1. Zusammensetzung in Form einer Öl-in-Wasser-Emulsion, umfassend:
a) eine kontinuierliche wässrige Phase,
b) eine in der wässrigen Phase dispergierte ölige Phase,
c) mindestens eine Tensidmischung, umfassend:
i) einen oder mehrere Fettsäureester eines Polyalkylenglykols,
ii) einen oder mehrere Fettsäureester von Glycerin,
iii) eine oder mehrere Fettsäuren in freier Form,
iv) ein oder mehrere Alkalimetallsalze eines Esters von Phosphorsäure und einem Fettalkohol und
d) eine oder mehrere UV-Filtersubstanzen;
wobei die Zusammensetzung bei einer Temperatur von 25 °C eine Viskosität kleiner oder gleich 0,10 Pa*s aufweist, wobei die Viskosität unter Verwendung eines Rheomat 180, der mit einer entsprechend der Konsistenz der Zusammensetzung ausgewählten MS-R1-, MS-R2-, MS-R3-, MS-R4- oder MS-R5-Spindel ausgestattet ist, die sich mit einer Drehzahl von 200 U/min dreht, gemessen wird, und
die Zusammensetzung keine die wässrige Phase verdickenden oder gelierenden Polymere umfasst und
wobei:
- es sich bei den Fettsäuren in den Fettsäureestern eines Polyalkylenglykols i), den Fettsäurenestern von Glycerin ii) und den Fettsäuren in freier Form iii) unabhängig voneinander um gesättigte oder ungesättigte Fettsäuren mit 12 bis 30 Kohlenstoffatomen handelt und
- der bzw. die Fettalkohol(e) in den Alkalimetallsalzen eines Esters von Phosphorsäure und einem Fettalkohol iv) 14 bis 36 Kohlenstoffatome umfasst bzw. umfassen.

2. Zusammensetzung nach Anspruch 1, **dadurch gekennzeichnet, dass** es sich bei dem Fettsäureester von Polyethylenglykol um einen Ester von Polyethylenglykol mit 1 bis 200 Oxyethylengruppen, vorzugsweise 1 bis 100 Oxyethylengruppen, und einer gesättigten oder ungesättigten Fettsäure mit 14 bis 26 Kohlenstoffatome handelt.

3. Zusammensetzung nach Anspruch 1 oder 2, **dadurch gekennzeichnet, dass** der Fettsäureester von Polyethylenglykol aus Polyethylenglykol-2-OE-monostearat oder Polyethylenglykol-100-monostearat ausgewählt ist.

4. Zusammensetzung nach einem der vorhergehenden Ansprüche, **dadurch gekennzeichnet, dass** es sich bei dem Fettsäureester von Polyethylenglykol um PEG-100-Stearat handelt.

5. Zusammensetzung nach einem der vorhergehenden Ansprüche, **dadurch gekennzeichnet, dass** es sich bei dem Fettsäureester von Glycerin um einen Ester einer gesättigten oder ungesättigten Fettsäure mit 14 bis 26 Kohlenstoffatomen und 1 bis 20 Glycerylgruppen und vorzugsweise 1 bis 10 Glyceryleinheiten handelt.

6. Zusammensetzung nach einem der vorhergehenden Ansprüche, **dadurch gekennzeichnet, dass** der Fettsäureester von Glycerin aus Glycerylmonostearat, Glycerylisostearat, Diglyceryldistearat, Tetraglyceryltristearat, Decaglyceryldecastearat, Diglycerylmonostearat, Hexaglyceryltristearat, Decaglycerylpentastearat, dem Ester von Glycerin und Palmitin- und Stearinsäure, Glycerylmono- und -dibehenat und Mischungen davon ausgewählt ist.

7. Zusammensetzung nach Anspruch 6, **dadurch gekennzeichnet, dass** der Fettsäureester von Glycerin aus Glycerylmonostearat, Glycerylisostearat und Mischungen davon ausgewählt ist.

8. Zusammensetzung nach einem der vorhergehenden Ansprüche, **dadurch gekennzeichnet, dass** die Fettsäure aus Laurinsäure, Myristinsäure, Palmitinsäure, Stearinsäure, Arachinsäure, Cetylsäure, Cetearylsäure und Behensäure ausgewählt ist.

9. Zusammensetzung nach einem der vorhergehenden Ansprüche, **dadurch gekennzeichnet, dass** es sich bei der Fettsäure in freier Form um Stearinsäure handelt.

10. Zusammensetzung nach einem der vorhergehenden Ansprüche, **dadurch gekennzeichnet, dass** die Alkalimetallsalze von Estern von Phosphorsäure und einem Fettalkohol aus den Natrium- und Kaliumsalzen von Monocetylphosphat, den Alkalimetallsalzen von Dicetylphosphat und den Alkalimetallsalzen von Dimyristylphosphat ausgewählt sind.

11. Zusammensetzung nach einem der vorhergehenden Ansprüche, **dadurch gekennzeichnet, dass** es sich bei dem Alkalimetallsalz eines Esters von Phosphorsäure und einem Fettalkohol um das Kaliumsalz von Monocetylphosphat handelt.

12. Zusammensetzung nach einem der vorhergehenden Ansprüche, **dadurch gekennzeichnet, dass** die Viskosität der Zusammensetzung bei einer Temperatur von 25 °C kleiner oder gleich 0,08 Pa*s und noch weiter bevorzugt kleiner 0,06 Pa*s ist.

13. Zusammensetzung nach einem der vorhergehenden Ansprüche, **dadurch gekennzeichnet, dass** es sich bei dem bzw. den Filtersubstanz(en) um UV-Filtersubstanzen in freier Form, die aus hydrophilen, lipophilen oder unlöslichen organischen UV-Filtersubstanzen und/oder einem oder mehreren anorganischen Pigmenten ausgewählt sind, handelt.

14. Zusammensetzung nach einem der vorhergehenden Ansprüche, **dadurch gekennzeichnet, dass** sie ein physiologisch unbedenkliches Medium und weiter bevorzugt ein kosmetisch unbedenkliches Medium umfasst.

15. Verwendung der Tensidmischung c) gemäß Anspruch 1 zur Stabilisierung einer Zusammensetzung in Form einer Öl-in-Wasser-Emulsion, die eine oder mehrere UV-Filtersubstanzen enthält.

16. Zusammensetzung nach einem der Ansprüche 1 bis 14 zur Verwendung bei einem Verfahren zur Begrenzung der Dunkelfärbung der Haut und/oder Verbesserung der Farbe und/oder Gleichmäßigkeit des Teints.

17. Zusammensetzung nach einem der Ansprüche 1 bis 14 zur Verwendung bei einem Verfahren zur Prävention und/oder Behandlung von Zeichen der Alterung eines Keratinmaterials, umfassend das Aufbringen.

## Revendications

1. Composition sous forme d'émulsion huile-dans-eau comprenant :
a) une phase aqueuse continue,
b) une phase huileuse dispersée dans ladite phase aqueuse,
c) au moins un mélange de tensioactifs comprenant :
i) un ou plusieurs esters d'acide gras et de polyalkylèneglycol,
ii) un ou plusieurs esters d'acide gras et de glycérol,
iii) un ou plusieurs acides gras sous forme libre,
iv) un ou plusieurs sels alcalins d'ester d'acide phosphorique et d'alcool gras, et
d) un ou plusieurs filtres UV ;
ladite composition présente une viscosité inférieure ou égale à 0,10 Pa.s à une température de 25°C, la viscosité étant mesurée à l'aide d'un Rhéomat 180, équipé d'un mobile MS-R1, MS-R2, MS-R3, MS-R4 ou MS-R5 choisi en fonction de la consistance de la composition, tournant à un vitesse de rotation de 200 t.min⁻¹ ; et
ladite composition ne comprenant pas de polymère épaississant ou gélifiant de la phase aqueuse ; et
dans laquelle :
- lesdits acides gras des esters d'acide gras et de polyalkylèneglycol i), des esters d'acide gras et de glycérol ii) et des acides gras sous forme libre iii) sont, indépendamment les uns des autres, des acides gras, saturés ou insaturés, comprenant de 12 à 30 atomes de carbone, et
- le(s)dit(s) alcool(s) gras des sels alcalins d'ester d'acide phosphorique et d'alcool gras iv) comprennent de 14 à 36 atomes de carbone.

2. Composition selon la revendication 1, **caractérisée en ce que** l'ester de polyéthylène glycol et d'acides gras est un ester de polyéthylène glycol comprenant de 1 à 200 groupes oxyéthylénés, de préférence de 1 à 100 groupes oxyéthylénés, et d'acide gras saturé ou insaturé comprenant de 14 à 26 atomes de carbone.

3. Composition selon la revendication 1 ou 2, **caractérisée en ce que** l'ester de polyéthylène glycol et d'acide gras est choisi parmi le monostéarate de polyéthylène glycol 2 OE ou le monostérate de polyéthylène glycol 100.

4. Composition selon l'une quelconque des revendications précédentes, **caractérisée en ce que** l'ester de polyéthylène glycol et d'acide gras est le stéarate de PEG-100.

5. Composition selon l'une quelconque des revendications précédentes, **caractérisée en ce que** l'ester d'acide gras et de glycérol est un ester d'acide gras saturés ou insaturés comprenant de 14 à 26 atomes de carbone, et de 1 à 20 groupes glycéryle et de préférence de 1 à 10 unités glycéryle.

6. Composition selon l'une quelconque des revendications précédentes, **caractérisée en ce que** l'ester d'acide gras et de glycérol est choisi parmi le mono stéarate de glycéryle, l'isostérate de glycéryle, le distéarate de diglycéryle, le tristéarate de tétraglycéryle, le décastéarate de décaglycéryle, le monostéarate de diglycéryle, le tristéarate d'hexaglycéryle, le pentastéarate de décaglycéryle, l'ester de glycérol et d'acides palmitique et stéarique, le mono et dibéhénate de glycéryle et leurs mélanges.

7. Composition selon la revendication 6, **caractérisée en ce que** l'ester d'acide gras et de glycérol est choisi parmi le monostérate de glycéryle, l'isostérate de glycéryle et leurs mélanges.

8. Composition selon l'une quelconque des revendications précédentes, **caractérisée en ce que** l'acide gras est choisi parmi l'acide laurique, l'acide myristique, l'acide palmitique, l'acide stéarique, l'acide arachidique, l'acide cétylique, l'acide cétéarylique, et l'acide béhénique.

9. Composition selon l'une quelconque des revendications précédentes, **caractérisée en ce que** l'acide gras sous forme libre est l'acide stéarique.

10. Composition selon l'une quelconque des revendications précédentes, **caractérisée en ce que** les sels alcalins d'esters d'acide phosphorique et d'alcool gras sont choisis parmi les sels de sodium et de potassium du monocétyle phosphate, les sels alcalins du dicétylphosphate et les sels alcalins du dimyristylphosphate.

11. Composition selon l'une quelconque des revendications précédentes, **caractérisée en ce que** le sel alcalin d'ester d'acide phosphorique et d'alcool gras est le sel de potassium du monocétyle phosphate.

12. Composition selon l'une quelconque des revendications précédentes, **caractérisée en ce que** la viscosité de la composition est inférieure ou égale à 0,08 Pa.s, et de façon encore plus préférée inférieure à 0,06 Pa.s à une température de 25°C.

13. Composition selon l'une quelconque des revendications précédentes, **caractérisée en ce que** le ou les filtres sont des filtres UV sous forme libre choisis parmi les filtres UV organiques hydrophiles, lipophiles ou insolubles et/ou un ou plusieurs pigments minéraux.

14. Composition selon l'une quelconque des revendications précédentes, **caractérisée en ce qu'**elle comprend un milieu physiologiquement acceptable et, plus préférentiellement, cosmétiquement acceptable.

15. Utilisation du mélange de tensioactifs c) tel que défini dans la revendication 1 pour stabiliser une composition sous forme d'émulsion huile-dans-eau contenant un ou plusieurs filtres UV.

16. Composition selon l'une quelconque des revendications 1 à 14 pour son utilisation pour limiter l'assombrissement de la peau et/ou améliorer la couleur et/ou l'homogénéité du teint.

17. Composition selon l'une quelconque des revendications 1 à 14 pour son utilisation dans un procédé pour prévenir et/ou traiter les signes du vieillissement d'une matière kératinique, comprenant l'application.
